# EUROPÄISCHE PATENTANMELDUNG

(11) **EP 0 816 341 A1**
(43) Veröffentlichungstag der Anmeldung: **07.01.1998**
(21) Anmeldenummer: 97110942.6
(22) Anmeldetag: 02.07.1997
(51) Int. Cl.: C07D 233/72, C07C 69/608, C07C 69/732, C07C 309/73, C07C 309/65

(54) **Verfahren zur Herstellung von chiralen Bernsteinsäurederivaten**

(30) Priorität: 04.07.1996 EP 96110814; 10.01.1997 EP 97100301
(71) Anmelder: F. HOFFMANN-LA ROCHE AG, 4070 Basel (CH)
(72) Erfinder: Brown, Paul Anthony, Hitchin SG4 9RW, Hertfordshire (GB); Hilpert, Hans, 4153 Reinach (CH)
(74) Vertreter: Mezger, Wolfgang, Dr.

(57) **Zusammenfassung**

Die vorliegende Erfindung betrifft ein neues Verfahren zur Herstellung einer Verbindung der Formel I 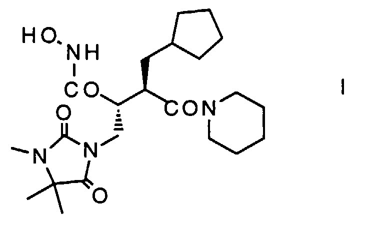 sowie neue Zwischenprodukte, die für dessen Herstellung geeignet sind.

## Beschreibung

Die vorliegende Erfindung betrifft ein Verfahren zur Herstellung einer Verbindung der Formel I 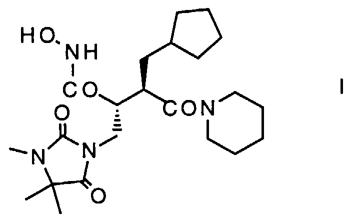 gekennzeichnet durch die folgenden Schritte:
a) Umsetzung einer Verbindung der Formel II 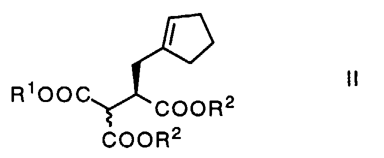 worin R² eine hydrogenolytisch abspaltbare Gruppe und
   R¹ Methyl, Ethyl, Propyl, Butyl, Pentyl, Hexyl oder t-Butyl bedeutet,
   mit 3-Brommethyl-1,5,5-trimethylhydantoin in Gegenwart einer Base zu einer Verbindung der Formel III 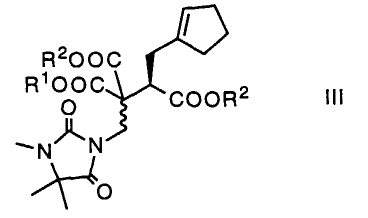 worin R¹ und R² die Bedeutungen wie vorstehend definiert haben,
b) Hydrogenolyse der Verbindung III in Gegenwart eines Metallkatalysators, nachfolgende Decarboxylierung in Gegenwart einer Base und Reinigung des so erhaltenen Produktes durch Zugabe einer weiteren Base, die zur Salzbildung geeignet ist, wobei eine Verbindung der Formel IV 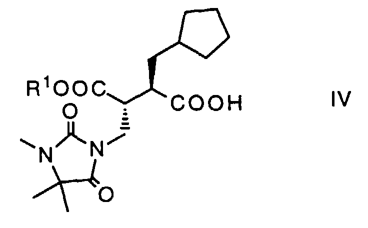 als Salz erhalten wird, worin R¹ wie oben definiert ist,
c) Umsetzung einer Verbindung der Formel IV mit Piperidin unter Aktivierung der Carbonsäure zu einer Verbindung der Formel V 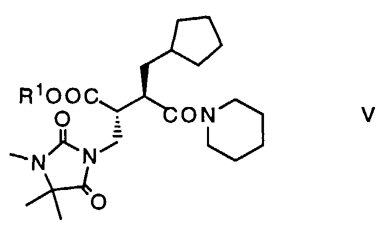 worin R¹ die oben angegebene Bedeutung hat,
d) Umsetzung einer Verbindung der Formel V,
   worin R¹ Methyl, Ethyl, Propyl, Butyl, Pentyl oder Hexyl bedeuten,
   mit einem mittels eines Alkylmagnesiumhalogenids aktivierten Benzylhydroxylaminhydrochlorids zur Verbindung VII 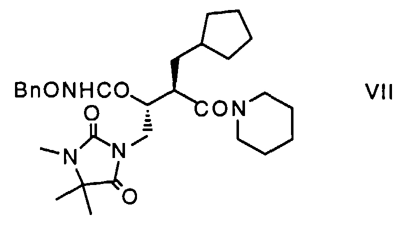 und hydrogenolytische Abspaltung der Benzylgruppe zur Verbindung I, oder
e) Abspaltung der t-Butylgruppe einer Verbindung der Formel V, in der R¹ tert-Butyl bedeutet, mit einer Mischung aus einer Mineralsäure und einer Carbonsäure und/oder einem Carbonsäureester,
   beziehungsweise Abspaltung der Estergruppe einer Verbindung der Formel V, in der R¹ Methyl, Ethyl, Propyl, Butyl, Pentyl oder Hexyl bedeutet, mit einem Alkali- oder Erdalkalimetallhydroxid
   zur Verbindung VI 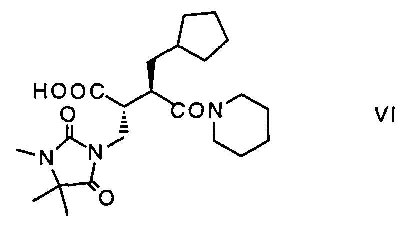 und entweder
   - anschliessende Umsetzung der Verbindung VI mit Benzylhydroxylaminhydrochlorid unter Zusatz eines Aktivierungsmittels zur Verbindung VII, und hydrogenolytische Abspaltung der Benzylgruppe in der Verbindung VII, wobei Verbindung I erhalten wird, oder
   - Umsetzung der Verbindung VI mit Trimethylsilyl-hydroxylamin oder Tetrahydropyranyl-hydroxylamin und Abspaltung der Trimethylsilyl- bzw. Tetrahydropyranyl-Gruppe nach an sich bekannten Methoden, wobei ebenfalls Verbindung I erhalten wird.

Die Verbindung I ist bekannt und beispielsweise in EP 684 240 A1 beschrieben. Die Verbindung hat wertvolle pharmakologische Eigenschaften und kann daher zur Behandlung und Vorbeugung von Krankheiten verwendet werden.

Gemäss der vorliegenden Erfindung kann eine Verbindung der Formel I u.a. durch die Reinigung eines Zwischenproduktes via Salzbildung und einer besseren Abspaltung der gewählten Estergruppen in einer reineren Form und in einer höheren Ausbeute als nach dem im Stand der Technik beschriebenen Verfahren erhalten werden.

Der Begriff "hydrogenolytisch abspaltbare Gruppe" in R² bedeutet eine Gruppe, die unter Anwendung von dem Fachmann bekannten Methoden, wie etwa unter Anwendung von Wasserstoff und einem Edelmetallkatalysator, abgespalten werden kann. In dieser Weise abspaltbare Estergruppen sind z.B. Phenacylester, Diphenylmethylester, p-Methoxybenzylester oder Benzylester. Vorzugsweise ist R² in einer der vorstehend bzw. noch nachfolgend beschriebenen Verbindungen Benzyl.

Der Ausdruck "nieder" bezeichnet Reste mit höchstens 7, vorzugsweise höchstens 4 Kohlenstoffatomen. Der Ausdruck "Alkyl" bezeichnet geradkettige oder verzweigte, gesättigte Kohlenwasserstoffreste, wie Methyl, Aethyl, Propyl, Isopropyl, n-Butyl und t-Butyl.

Alle Temperaturangaben sind in Grad Celcius.

Die Alkylierung des Triesters II mit dem 3-Brommethyl-1,5,5-trimethylhydantoin erfolgt dadurch, dass der Triester zunächst mit einer Base, wie einem Alkali- oder Erdalkalimetallhydroxid oder -alkoxid oder einem Metallhydrid, vorzugsweise Natriumhydrid, in einem Lösungsmittel wie einem Ether, z.B. 1,2-Dimethoxyethan, oder einem Sulfoxid, z.B. Dimethylsulfoxid, oder einem Ester, z.B. Essigester, oder einem Formamid, vorzugsweise Dimethylformamid, bei einer Temperatur von 0° bis 100°, vorzugsweise 10° bis 30°, deprotoniert wird, und das Hydantoin mit dem Triester II im gleichen Lösungsmittel umgesetzt wird. Zweckmässigerweise wird das Metallmalonat zum 3-Brommethyl-1,5,5-trimethylhydantoin bei einer Temperatur von -20° bis 50°, vorzugsweise bei 0° bis 10°, gegeben.

Das 3-Brommethyl-1,5,5-trimethylhydantoin für die Reaktion mit dem Triester II kann durch Brommethylierung von 1,5,5-Trimethylhydantoin erhalten werden. Dazu wird zweckmässigerweise 1,5,5-Trimethylhydantoin mit Bromwasserstoff in Essigsäure bei einer Temperatur zwischen 20° und 100°, vorzugsweise bei etwa 80° umgesetzt. Das Trimethylhydantoin ist nach an sich bekannten Methoden erhältlich (H. Heimgartner et al., Helv. Chim. Acta 75, 1251 (1992)).

Eine Verbindung der Formel II kann nach der unten beschriebenen Methode erhalten werden.

Die Debenzylierung und Hydrierung der Doppelbindung des nach Alkylierung mit dem Hydantoin erhaltenen Triesters III erfolgt in Gegenwart der üblichen Edelmetallkatalysatoren wie z.B. Palladium oder Platin, insbesondere Palladium, zweckmässig auf Trägern wie Alox, Bariumsulfat oder Kohle, bevorzugt ist die Verwendung von z.B. 5% Pd auf Kohle, in Gegenwart eines Lösungsmittels wie einem Kohlenwasserstoff, z.B. Toluol, oder einem halogenierten Kohlenwasserstoff, wie Methylenchlorid oder einem Ester, wie Essigester, oder einem Ether, wie t-Butylmethylether, oder vorzugsweise in einem Alkohol, besonders bevorzugt ist i-Propanol, bei einer Temperatur von 20° bis 100°, bevorzugt 20 bis 30°.

Die nachfolgende Decarboxylierung erfolgt ohne Isolierung der intermediär gebildeten Disäure in Gegenwart einer Base, wie z.B. einem Alkylamin, vorzugsweise einem Trialkylamin wie N-Methylmorpholin, und einem aprotischen Lösungsmittel wie einem Kohlenwasserstoff, z.B. Toluol, oder einem halogenierten Kohlenwasserstoff, wie Chloroform, oder einem Ether, wie Diisopropylether, oder einem Ester wie Essigester oder bevorzugt Isopropylacetat bei einer Temperatur von 40° bis 140°, bevorzugt bei 80° bis 90°. Die kombinierte Verwendung von N-Methylmorpholin als Base und Isopropylacetat als Lösungsmittel liefert ein besonders gutes Isomerenverhältnis von (2R,3R):(2R,3S) von 85:15.

Die Reinigung der Säure erfolgt durch Salzbildung unter Zugabe eines weiteren Alkylamins, besonders bevorzugt mit (+)-Pseudoephedrin oder Ethanolamin, im gleichen Lösungsmittel wie die Decarboxylierung durchgeführt wird. Dabei fällt ein chemisch und optisch reines Material der Formel IV als Salz an.

Für die Umsetzung mit Piperidin wird das Salz zunächst in die freie Säure überführt, indem es zwischen einem Lösungsmittel, bevorzugt ein Lösungsmittel, das auch für die nachfolgende Kopplung geeignet ist wie z.B. einem Ester, bevorzugt Essigester, und einer Mineralsäure wie z.B. Salzsäure verteilt wird. Die organische Phase wird getrocknet und für die Amidbildung eingesetzt.

Die Amidbildung der Säure in einer Verbindung der Formel IV mit Piperidin, unter Aktivierung der Carbonsäure, zu einer Verbindung der Formel V kann nach an sich bekannten Kopplungsmethoden, wie z.B. via Säurechlorid, via gemischtes Anhydrid, via einen Aktivester, oder bevorzugt via gemischtes Sulfonsäureanhydrid erfolgen. Dabei werden wasserziehende Mittel wie ein Sulfonsäurehalogenid, bevorzugt Methansulfochlorid, oder ein Carbodiimid, bevorzugt Dicyclohexyl-carbodiimid, in Gegenwart von stöchiometrischen oder katalytischen Mengen an Aktivestern bildenden Alkoholen, wie z.B. N-Hydroxysuccinimid, N-Hydroxybenzotriazol oder bevorzugt N-Hydroxy-2-pyridon, in einem Lösungsmittel wie einem Keton, z.B. Methylethylketon, oder einem Ether, z.B. t-Butylmethylether, oder einem Kohlenwasserstoff, z.B. Toluol, oder einem halogenierten Kohlenwasserstoff, z.B. Methylenchlorid oder einem Ester bevorzugt Essigester, bei einer Temperatur von 0 bis 80°, bevorzugt 10 bis 25°, eingesetzt.

Die direkte Überführung eines Esters mit der Formel V worin R¹ Methyl, Ethyl, Propyl, Butyl, Pentyl oder Hexyl, vorzugsweise Methyl, bedeutet, in das Benzylhydroxamat VII erfolgt durch Aktiviervng des O-Benzylhydroxylaminhydrochlorids mit einem Alkylmagnesiumhalogenid, bevorzugt i-Propylmagnesiumchlorid, in Gegenwart des Esters V, in einem Lösungsmittel wie einem Ether, z.B. t-Butylmethylether oder, bevorzugt THF, bei einer Temperatur von -70° bis 50°, bevorzugt -20° bis 0°.

Die Abspaltung der t-Butylgruppe einer Verbindung der Formel V, in der R¹ tert-Butyl bedeutet, zur Verbindung VI erfolgt in Gegenwart einer Mineralsäure, wie z.B. wässriger Phosphor- oder Schwefelsäure, bevorzugt Salzsäure oder Bromwasserstoffsäure und einer organischen Carbonsäure, bevorzugt Essigsäure bei einer Temperatur von 0 bis 100°, bevorzugt 0 - 22°. Statt in einer Carbonsäure kann die Abspaltung auch in einem Ester einer Carbonsäure oder einem Gemisch von Carbonsäure und Carbonsäureester erfolgen. Geeignete Carbonsäureester sind Essigsäuremethyl, -ethyl oder -isopropylester, bevorzugt Essigsäureethylester. Als bevorzugte Abspaltungsmethode wird HBr in einem Gemisch von Essigsäure/Essigsäureethylester verwendet. Weiterhin kann die Abspaltung mittels einer Säure auch in einem sonstigen geeigneten organischen Lösungsmittel erfolgen. Geeignete organische Lösungsmittel sind Methylenchlorid oder Toluol.

Die Hydrolyse einer Estergruppe einer Verbindung der Formel V, in der R¹ Methyl, Ethyl, Propyl, Butyl, Pentyl oder Hexyl, vorzugsweise Methyl, bedeutet, zur Verbindung VI erfolgt in Gegenwart eines Alkali- oder Erdalkalimetallhydroxids wie Barium-, Kalzium-, Natrium- oder Kaliumhydroxid, bevorzugt Kaliumhydroxid, in einem Lösungsmittel wie einem Alkohol, z.B. i-Propanol oder Wasser mit einem organischen Lösungsmittel wie einem Ether, z.B. t-Butylmethylether, oder bevorzugt THF, bei einer Temperatur von 0 bis 100°, bevorzugt 30 bis 50°. Vorzugsweise wird bei einer der Verbindungen der Formel II - V der t-Butylester verwendet.

Die Umwandlung der Verbindung VI in das Benzylhydroxamat VII erfolgt unter Verwendung von Benzylhydroxylaminhydrochlorid und einem Aktivierungsmittel in analoger Weise wie es oben für die Amidbildung der Säure mit Piperidin beschrieben ist. Besonders bevorzugte Aktivierungsmittel sind Carbodiimide, z.B. Dicyclohexylcarbodiimid oder ein Isocyanid, z.B. t-Butylisocyanid, bevorzugt 2-Morpholino-ethylisocyanid, in Gegenwart von stöchiometrischen oder katalytischen Mengen an Aktivestern bildenden Alkoholen, wie z.B. N-Hydroxysuccinimid, N-Hydroxybenzotriazol oder bevorzugt N-Hydroxy-2-pyridon. Die Anwendung und Herstellung solcher Isocyanide ist in EP 29 909 B1 beschrieben.

Die Debenzylierung der Verbindung VII zu Verbindung I erfolgt in einem organischen Lösungsmittel unter Verwendung von Wasserstoff in Gegenwart eines Metallkatalysators. Geeignete Lösungsmittel sind C₁-C₆-Alkohole, bevorzugt Methanol oder Ethanol. Als Metallkatalysatoren können Platin oder Palladium verwendet werden, die sich zweckmässigerweise auf einem Trägermaterial wie Aluminiumoxid, Bariumsulfat oder Kohle befinden. Ein bevorzugter Katalysator ist Palladium auf Kohle oder Bariumsulfat. Temperatur und Druck sind nicht kritisch und können in einem weiten Bereich gewählt werden. Vorzugsweise wird die Hydrierung bei Raumtemperatur und 1-10 bar durchgeführt.

Die Einführung der Hydroxylamingruppe mittels O-Trimethylsilylhydroxylamin erfolgt mit an sich bekannten Aktivierungsmitteln wie Carbodiimiden, z.B. Dicyolohexylcarbodiimid oder einem Isocyanid, z.B. t-Butylisocyanid, bevorzugt 2-Morpholino-ethylisocyanid in Gegenwart von stöchiometrischen oder katalytischen Mengen an Aktivestern bildenden Alkoholen, wie z.B. N-Hydroxysuccinimid, N-Hydroxybenzotriazol oder bevorzugt N-Hydroxy-3-pyridon, in einem Lösungsmittel wie einem Ether, z.B. t-Butylmethylether, oder einem Kohlenwasserstoff, z.B. Toluol, oder einem halogenierten Kohlenwasserstoff oder einem Ester bevorzugt Methylenchlorid bzw. Essigester bei einer Temperatur von 0 bis 80°, bevorzugt 10 bis 25°. Unerwarteterweise wurde gefunden, dass bei der wässrigen Aufarbeitung die TMS-Schutzgruppe glatt gespalten wird und das Zielprodukt I ohne Isolierung des TMS-geschützten Zwischenproduktes in hoher Ausbeute und Reinheit erhalten werden kann.

In einer bevorzugten Ausführungsform erfolgt die Herstellung von Verbindung I aus Verbindung V über die Verbindung VI, d.h. nicht über den Schritt d) sondern die Alternative e) mit R¹ gleich t-Butyl, gefolgt von der anschliessenden Umsetzung der Verbindung VI mit Trimethylsilylhydroxylamin.

Die vorliegende Erfindung betrifft weiterhin ein Verfahren zur Herstellung einer Verbindung der Formel II, die als Edukt für die Synthese der Verbindung I, eingesetzt werden kann. In einer bevorzugten Ausführungsform werden dazu die beiden Verfahren zur Herstellung der Verbindungen I und II gemäss der vorliegenden Erfindung kombiniert.

Das Verfahren zur Herstellung einer Verbindung der Formel II 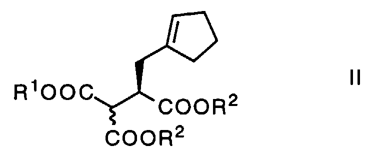 worin
R¹ Methyl, Ethyl, Propyl, Butyl, Pentyl, Hexyl oder tert-Butyl und
R² eine hydrogenolytisch abspaltbare Gruppe bedeuten,
ist gekennzeichnet durch die folgenden Schritte:
a) Umsetzung eines Glyoxylsäurederivates der Formel VIII 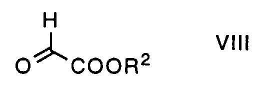 mit Methylencyclopentan in Gegenwart von katalytischen Mengen eines (R)-Binaphthyloxytitanium-Katalysators zu einer Verbindung der Formel IX 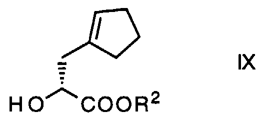 wobei R² eine hydrogenolytisch abspaltbare Gruppe darstellt,
b) Aktivierung einer Verbindung der Formel IX mit einem Sulfonsäurehalogenid R³-SO₂-X, worin R³ Trifluormethyl oder gegebenenfalls durch Nitro oder Halogen substituiertes Phenyl, und X Halogen bedeuten,
   zu einer Verbindung der Formel X 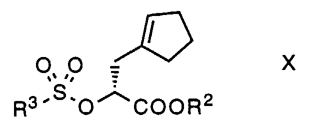 worin R² und R³ die vorstehend angegebenen Bedeutungen haben, und
c) Umsetzung einer Verbindung der Formel X mit einem Malonsäurederivat der Formel XI in Gegenwart einer Base

   R¹O₂CCH₂CO₂R² XI

   worin R² die vorstehende Bedeutung hat und
   R¹ Methyl, Ethyl, Propyl, Butyl, Pentyl, Hexyl oder t-Butyl bedeutet,
   zu einer Verbindung der Formel II.

Der Begriff "Binaphthyloxytitanium-Katalysator" bedeutet einen Katalysator dessen weitere Liganden am Titan des Binaphthyloxytitan-Kerns aus der Gruppe der Halogenide, vorzugsweise Chlor oder Brom, stammen oder dessen weiterer Ligand nochmals eine Binaphthyloxytitan-Einheit sein kann. Solche Katalysatoren sind in EP 353 053 A2 und EP 676 404 A2 unter Formel (IV) bzw. (I) beschrieben.

Vorzugsweise erfolgt die Glyoxylat-en-Reaktion einer Verbindung der Formel VIII mit Methylencyclopentan zum Hydroxyester IX mit in situ erzeugtem (R)-Binaphthyloxytitandibromid oder -dichlorid nach an sich bekannter Methode [Mikami et al. in J. Am. Chem. Soc. 112, 1990, 3949-3954] in einem Lösungsmittel wie einem Keton, z.B. Methylethylketon, oder einem Ether, z.B. t-Butylmethylether, oder einem Kohlenwasserstoff, z.B. Toluol, oder einem halogenierten Kohlenwasserstoff, vorzugsweise Methylenchlorid, bei -70° bis 35°, vorzugsweise 10 bis 25°. Vorzugsweise wird ein Glyoxylatderivat der Formel VIII verwendet in dem R² Benzyl ist. Methylencyclopentan ist käuflich erhältlich oder kann in bekannter Weise hergestellt werden (J.M. Conia, J.C. Limasset, Bul. Soc. Chim. Fr. 1939, (1967)). Benzylglyoxylat ist ebenfalls in bekannter Weise erhältlich (J.E. Bishop, J.F. O'Connell, H. Rapoport, J. Org. Chem. 56, 5079 (1991)). Andere Glyoxylatderivate zur Anwendung in dem vorliegenden Verfahren können gemäss der Beschreibung in Synthesis S. 544 (1972) hergestellt werden.

Trotz Verwendung eines sterisch anspruchsvollen Substituenten in der R² Position konnte ein hoher Enantiomerenüberschuss für IX erzielt werden. Die im Stand der Technik beschriebenen Ergebnisse weisen eher auf die Verwendung von kleinen Resten wie etwa Methyl hin. Ferner wurde unerwarteterweise bei der vorliegenden Reaktion gefunden, dass der Enantiomerenüberschuss (ee) besonders hoch war (ca. 98%), wenn die Reaktion bei Raumtemperatur durchgeführt wird. Im Stand der Technik wurden für eine ähnliche Reaktion Temperaturen von unter 0°C angegeben bzw. von -30°C verwendet.

Für die Bildung des Sulfonates X kann isolierter Hydroxyester oder, zweckmässigerweise, direkt der in Lösung verbleibende Hydroxyester verwendet werden. Die Reaktion des Hydroxyesters mit einem Sulfonsäurehalogenid (R³-SO₂-X) erfolgt in Gegenwart einer Base, wie einem nieder-Alkylamin, vorzugsweise Triethylamin, in einem oben genannten Lösungsmittel, vorzugsweise Methylenchlorid, bei -70° bis +70°, vorzugsweise bei -20 bis -10°. Ein besonders bevorzugtes Sulfonsäurehalogenid ist o-Nitrobenzolsulfochlorid, dessen Ester in hoher optischer Reinheit (>99.9%) kristallisiert werden kann, insbesondere wenn bei der Reaktion der Hydroxybenzylester verwendet wird.

Die Alkylierung des Sulfonates X mit dem Malonat (R¹O₂C-CH₂-COOR²) XI wird dadurch bewerkstelligt, dass das Malonat zunächst mit einer Base, wie einem Alkali- oder Erdalkalimetallhydroxid oder -alkoxid oder einem Metallhydrid, vorzugsweise Natriumhydrid, in einem Lösungsmittel wie einem Ether, z.B. 1,2-Dimethoxyethan, oder einem Sulfoxid, z.B. Dimethylsulfoxid, oder einem Ester, z.B. Essigester, oder einem Formamid, vorzugsweise Dimethylformamid, bei einer Temperatur von 0° bei 100°, Vorzugsweise 10 bis 25°, deprotoniert wird, und das Metallmalonat mit dem Sulfonat im gleichen Lösungsmittel bei einer Temperatur von 0° bis 100°, vorzugsweise bei 10 bis 20° umgesetzt wird. Die Malonatderivate selbst sind in an sich bekannter Weise durch schrittweise Derivatisierung von Malonsäure erhältlich. Vorzugsweise ist im Malonat der Formel XI R¹ gleich t-Butyl.

Die neuen Zwischenverbindungen sind ebenfalls Gegenstand der vorliegenden Erfindung. Insbesondere sind dies:
Verbindungen der Formel II, insbesondere
   (2R,3R)- und (2S,3R)-2-tert-Butyloxycarbonyl-3-cyclopent-1-enylmethylbernsteinsäuredibenzylester und
   (2R,3R)- und (2S,3R)-2-Methyloxycarbonyl-3-cyclopent-1-enylmethylbernsteinsäuredibenzylester;
Verbindungen der Formel III, insbesondere
   (2R,3R)- und (2S,3R)-2-tert-Butoxycarbonyl-3-cyclopent-1-enylmethyl-2-(3,4,4-trimethyl-2,5-dioxo-imidazolidin-1-yl-methyl)-bernsteinsäuredibenzylester und
   (2R,3R)- und (2S,3R)-2-Methyloxycarbonyl-3-cyclopent-1-enylmethyl-2-(3,4,4-trimethyl-2,5-dioxo-imidazolidin-1-yl-methyl)-bernsteinsäuredibenzylester;
Verbindungen der Formel IV, insbesondere
   4-tert-Butyl-2(R)-(cyclopentylmethyl)-3(R)-[(3,4,4-trimethyl-2,5-dioxo-1-imidazolidinyl)methyl]-bernsteinsäure und
   4-Methyl-2(R)-(cyclopentylmethyl)-3(R)-(3,4,4-trimethyl-2,5-dioxo-1-imidazolidinyl)methyl]-bernsteinsäure und deren Salze mit (+) - Pseudoephedrin oder Ethanolamin;
Verbindungen der Formel V, insbesondere
   1-[2(R)-[1(R)-(Methoxycarbonyl)-2-(3,4,4-trimethyl-2,5-dioxo-1-imidazolidinyl)ethyl]-3-cyclopentylpropionyl]piperidin und
   1-[2(R)-[1(R)-(tert-Butoxycarbonyl)-2-(3,4,4-trimethyl-2,5-dioxo-1-imidazolidinyl)ethyl]-3-cyclopentylpropionyl]piperidin;
1-[2(R)-[1(R)-Carboxy-2-(3,4,4-trimethyl-2,5-dioxo-1-imidazolidinyl)ethyl]-3-cyclopentylpropionyl)piperidin;
1-[2(R)-[1(R)-(Benzyloxycarbamoyl)-2-(3,4,4-trimethyl-2,5-dioxo-1-imidazolidinyl)ethyl]-3-cyclopentylpropionyl)piperidin;
Verbindungen der Formel IX, insbesondere (R)-3-Cyclopent-1-enyl-2-hydroxy-propionsäurebenzylester; und
Verbindungen der Formel X, insbesondere (R)-3-Cyclopent-1-enyl-2-(2-nitro-phenylsulfonyloxy)-propionsäurebenzylester.

Die Zwischenverbindungen der vorliegenden Erfindung können zur Herstellung einer Verbindung der Formel I bzw. II verwendet werden. Insbesondere geeignet sind Verbindungen der Formel II, V und IX.

Die folgenden Beispiele veranschaulichen die vorliegende Erfindung ohne sie zu beschränken.

### Beispiele

### Beispiel 1

Zu 11 ml Katalysatorsuspension bestehend aus 135 mg (R)-(+)-1,1'-Binaphthol, 153 mg Diisopropoxytitan (IV) dibromid, 0.9 ml Toluol, 8 ml Methylenchlorid und 2.8 g Molekularsieb (4 Å, Pulver, Aldrich), hergestellt nach K. Mikami et al., Org. Synth. 71, 14 (1993), wurde bei 22° 6.60 g Methylen-cyclopentan gegeben und anschliessend mit einer hitzebehandelten Lösung bestehend aus 18.55 g Benzylglyoxylat und 48 ml Toluol (Lösung zuerst 16 Std. am Rückfluss erhitzt und auf 22° abgekühlt) während 80 Min. versetzt. Die orange Suspension wurde 3 Std. gerührt, erneut mit 11 ml der oben beschriebenen Katalysatorsuspension versetzt und über Nacht bei +4° aufbewahrt. Die Suspension wurde filtriert, das Filtrat mit dem darin enthaltenen (R)-3-Cycopenten-1-enyl-2-hydroxy-propionsäurebenzylester mit 23.0 g o-Nitrobenzolsulfochlorid versetzt, die Lösung auf -15° abgekühlt und während 45 Min. mit 23.9 g Triethylamin behandelt. Nach 5 Std. bei -15° wurde mit 150 ml 1 N Schwefelsäure sauergestellt und mit Methylenchlorid extrahiert. Die Methylenchloridphasen wurden mit Wasser gewaschen, getrocknet und eingeengt. Der Rückstand wurde aus 170 ml Essigester umkristallisiert und das Kristallisat getrocknet, wobei man 19.2 g (55%) reinen (R)-3-Cyclopent-1-enyl-2-(2-nitro-phenylsulfonyloxy)-propionsäurebenzylester erhielt, Smp. 122.5-124.5°, erhielt. IR (KBr): 1736s (C=O), 1543s und 1370s (NO₂), 1380s und 1191s (SO₃).

### Beispiel 2

Eine Suspension von 21.3 g 1,5,5-Trimethylhydantoin, 5.86 g Paraformaldehyd und 34 ml Bromwasserstoff in Essigsäure (33%) wurde 2 Std. auf 80° erhitzt, erneut mit 7.9 ml Bromwasserstoff in Essigsäure behandelt und weitere 2.5 Std. erhitzt. Die Lösung wurde auf 0° gekühlt, mit 100 ml Methylenchlorid verdünnt und anschliessend bei 0° mit 100 ml eiskaltem Wasser versetzt. Die Methylenchloridphase wurde mit Wasser gewaschen, getrocknet und eingeengt. Der Rückstand wurde aus t-Butylmethylether/Hexan kristallisiert und das Kristallisat getrocknet, wobei man 30.9 g (88%) reines 3-Brommethyl-1,5,5-trimethylhydantoin, Smp. 86-88°, erhielt. IR (KBr): 1780s und 1732s (C=O).

### Beispiel 3

Eine Suspension von 3.17 g NaH (55-65% in Oel, zuerst mit Hexan gewaschen) in 30 ml DMF wurde mit einer Lösung von 18.02 Benzyl-t-butylmalonat in 30 ml DMF versetzt und 30 Min bei 22° gerührt. Die Lösung wurde portionenweise mit 31.06 g o-Nisylat aus Beispiel 1 behandelt, 6 Std. bei 22° gerührt, erneut 6.21 g o-Nisylat zugegeben und 22 Std. bei 22° gerührt. Die rote Lösung wurde bei 10° mit 100 ml Hexan und 200 ml Wasser verdünnt, 1 Std. bei 10° gerührt und überschüssiges o-Nisylat durch Filtration entfernt. Die organische Phase im Filtrat wurde mit Wasser gewaschen, getrocknet und eingeengt. Der Rückstand enthielt 35 g einer 1:1 Mischung des Triester (2R,3R)- und (2S,3R)-2-tert-Butyloxycarbonyl-3-cyclopent-1-enylmethyl-bernsteinsäuredibenzylester, der ohne weitere Reinigung in die nächste Stufe eingesetzt wurde.

### Beispiel 4

Analog Beispiel 3 wurde aus dem Sulfonat von Beispiel 1 durch Umsetzung mit Benzyl-Methylmalonat das 1:1 Gemisch des Triester (2R,3R)- und (2S,3R)-2-Methyloxycarbonyl-3-cyclopent-1-enylmethylbernsteinsäuredibenzylester erhalten.

### Beispiel 5

Eine Suspension von 3.15 g NaH (55-65% in Oel, zuerst mit Hexan gewaschen) in 30 ml DMF wurde mit einer Lösung von 34.95 g des Triestergemisches aus Bsp. 3 in 60 ml DMF versetzt und 30 Min bei 22° gerührt. Die entstandene Lösung wurde bei 0° zu einer Lösung bestehend aus 20.2 g 2-Brommethyl-1,5,5-trimethylhydantoin aus Bsp. 2 und 30 ml DMF getropft und 4 Std. bei 0° gerührt. Die Suspension wurde bei 0° mit 300 ml Cyclohexan und 300 ml Wasser verdünnt, die organische Phase wurde mit 0.1 N Salzsäure, mit 0.1 N Natronlange und mit Wasser gewaschen, getrocknet und eingeengt. Der Rückstand enthielt 47.3 g des Gemisches von (2R,3R)- und (2S,3R)-2-tert-Butoxycarbonyl-3-cyclopent-1-enylmethyl-2-(3,4,4-trimethyl-2,5-dioxo-imidazolidin-1-yl-methyl)-bernsteinsäuredibenzylester, das ohne weitere Reinigung in der nächsten Stufe eingesetzt wurde.

### Beispiel 6

Analog Beispiel 5 wurde aus dem Produkt von Bsp. 4 durch Umsetzung mit 2-Brommethyl-1,5,5-trimethylhydantoin das entsprechende 1:1 Gemisch von (2R,3R)- und (2S,3R)-2-Methyloxycarbonyl-3-cyclopent-1-enylmethyl-2-(3,4,4-trimethyl-2,5-dioxo-imidazolidin-1-yl-methyl)-bernsteinsäuredibenzylester erhalten.

### Beispiel 7

Eine Suspension bestehend aus 46.9 g des Hydantoyltriesters aus Bsp. 5 und 9.38 g Pd/C (5%) in 470 ml i-PrOH wurde bei 22-33°/1 bar während 24 h hydriert. Die Suspension wurde filtiert, das Filtrat vollständig eingeengt und der Rückstand in 460 ml i-Propylacetat gelöst. Die Lösung wurde mit 7.2 g N-Methylmorpholin versetzt, 4 Std. am Rückfluss erhitzt, mit 11.7 g (+)-Pseudoephedrin versetzt und auf 22° abgekühlt. Nach 4 Std. bei 22° wurde filtriert und das Kristallisat getrocknet, wobei man 24.5 g (60%) reines Ephedrinsalz von 4-tert-Butyl-2(R)-(cyclopentylmethyl)-3(R)-[(3,4,4-trimethyl-2,5-dioxo-1-imidazolidinyl)methyl]-bernsteinsäure, Smp. 169-170°, erhielt. IR (KBr): 3234 m, br. (NH, OH), 1771m und 1720s, br. (C=O).

### Beispiel 8

Analog Beispiel 7 wurde durch Umsetzung des Methylderivates aus Bsp. 6 als Produkt das Ephedrinsalz von 4-Methyl-2(R)-(cyclopentylmethyl)-[3(R)-(3,4,4-trimethyl-2,5-dioxo-1-imidazolidinyl)methyl]-bernsteinsäure, Smp. 141-143°, in 44-50% Ausbeute erhalten. IR (KBr): 3433m, br. (NH, OH), 1770m und 1735s und 1714s (C=O).

### Beispiel 9

Eine Lösung von 8.64 g Ephedrinsalz aus Bsp. 7 in 50 ml Essigester wurde mit 50 ml 2N Salzsäure geschüttelt, die organische Phase mit Wasser gewaschen und die wässrigen Phasen mit 25 ml Essigester nachextrahiert. Die vereinigten Essigesterphasen wurden getrocknet, nacheinander mit 0.32 g N-Hydroxy-2-pyridon, 1.22 g Piperidin und einer Lösung bestehend aus 3.24 g Dicyclohexylcarbodiimid und 17.5 ml Essigester bei 22° versetzt und 16 Std. gerührt. Die Suspension wurde 1 Std. bei 0° gerührt, abfiltriert, das Filtrat mit Natriumbikarbonat-Lösung und Wasser gewaschen, getrocknet und eingeengt. Der Rückstand enthielt 7.8 g 1-[2(R)-[1(R)-(tert-Butoxycarbonyl)-2-(3,4,4-trimethyl-2,5-dioxo-1-imidazolidinyl)ethyl]-3-cyclopentylpropionyl]piperidin, das ohne weitere Reinigung in die nächste Stufe eingesetzt wurde.

### Beispiel 10

Analog Beispiel 9 wurde durch Umsatz das Ephedrinsalzes von Bsp. 8 das Produkt 1-[2(R)-[1(R)-(Methoxycarbonyl)-2-(3,4,4-trimethyl-2,5-dioxo-1-imidazolidinyl)ethyl]-3-cyclopentylpropionyl]piperidin erhalten, das ohne weitere Reinigung in der nächsten Stufe eingesetzt wurde.

### Beispiel 11

Zu einer Lösung von 475 mg t-Butylester aus Bsp. 9 in 1.3 ml Essigsäure wurde 0.84 ml 37%ige Salzsäure gegeben und 1 Std. bei 22° gerührt. Die Lösung wurde mit 3 ml Wasser verdünnt und mit Methylenchlorid extrahiert. Die Extrakte wurden mit Wasser gewaschen, getrocknet und eingeengt. Der Rückstand enthielt 393 mg reines 1-[2(R)-[1(R)-Carboxy-2-(3,4,4-trimethyl-2,5-dioxo-1-imidazolidinyl)ethyl]-3-cyclopentylpropionyl) piperidin. IR (KBr): 1769m und 1714s (C=O).

Alternativ wurde zu einer Lösung von 8.30 g t-Butylester aus Bsp. 9 in 50 ml Essigester bei 0° 12.2 ml 33%ige Bromwasserstoffsäure in Essigsäure gegeben und 3 1/2 Std. bei 0° gerührt. Die organische Phase wurde mit Wasser neutral gewaschen, getrocknet und eingeengt. Der Rückstand enthielt 7.3 g reines 1-[2(R)-[1(R)-Carboxy-2-(3,4,4-trimethyl-2,5-dioxo-1-imidazolidinyl)ethyl]-3-cyclopentylpropionyl)piperidin.

### Beispiel 12

Eine Lösung von 1.78 g Methylester aus Bsp. 10 in 3 ml THF wurde mit einer Lösung von 0.69 g KOH in 6.1 ml Wasser versetzt, 5 Std. bei 0° und 10 Std. bei 40° kräftig gerührt. Das Gemisch wurde mit verdünnter Salzsäure auf pH=2 gestellt und mit 8 ml THF und 6 ml gesättigter Kochsalzlösung versetzt. Die THF-Phase wurde mit halbgesättigter Kochsalzlösung gewaschen, getrocknet und eingeengt. Der Rückstand enthielt 1.68 g ca. 95% reines 1-[2(R)-[1(R)-Carboxy-2-(3,4,4-trimethyl-2,5-dioxo-1-imidazolidinyl) ethyl]-3-cyclopentylpropionyl)piperidin VI. IR (KBr): 1769m und 1714s (C=O).

### Beispiel 13

Zu einer Lösung von 1.38 g 1-[2(R)-[1(R)-Carboxy-2-(3,4,4-trimethyl-2,5-dioxo-1-imidazolidinyl)ethyl]-3-cyclopentylpropionyl)piperidin VI in 13 ml Methylenchlorid wurde bei 0° nacheinander 0.74 g N-Ethylmorpholin, 0.60 g N-Hydroxybenzotriazol-hydrat und 0.75 g N-Ethyl-N'-(3-dimethylaminopropyl)-carbodiimid gegeben und 20 Min. bei 0° gerührt. Das Reaktionsgemisch wurde mit 0.45 g N-Ethylmorpholin und 0.63 g O-Benzylhydroxylamin-hydrochlorid versetzt, 30 Min. bei 0° und 17 Std. bei 22° gerührt. Die Lösung wurde mit 13 ml Methylenchlorid verdünnt, mit Natriumbikarbonatlösung und verdünnter Salzsäure gewaschen, getrocknet und eingeengt. Der Rückstand wurde aus Essigester/Hexan kristallisiert und das Kristallisat getrocknet, wobei man 1.26 g (73%) reines 1-[2(R)-[1(R)-(Benzyloxycarbamoyl)-2-(3,4,4-trimethyl-2,5-dioxo-1-imidazolidinyl)ethyl]-3-cyclopentylpropionyl)piperidin VII, Smp. 138-140°, erhielt.

Alternativ wurde zu einer Suspension von 10.54 g der Säure aus Bsp. 11 und 3.06 g N-Hydroxy-2-pyridon in 110 ml Methylenchlorid bei 22° 3.86 g 2-Morpholino-ethylisocyanid gegeben und 2 Std. gerührt. Die Lösung wurde mit 3.39 g O-Benzylhydroxylamin versetzt und 5 Std. gerührt. Das Reaktionsgemisch wurde mit verdünnter Salzsäure, NaHCO₃-Lösung und Wasser gewaschen, getrocknet und eingeengt. Der Rückstand liefert nach Umkristallisation aus Methylenchlorid/Hexan 11.19 g (85%) reines Benzylhydroxamat VII, Smp. 140-142°.

### Beispiel 14

Eine Lösung von 1.10 g Methylester aus Bsp. 10 und 568 mg O-Benzylhydroxylamin-hydrochlorid in 7 ml THF wurde bei -20° mit 3.5 ml einer 2 M i-PrMgCl-Lösung in THF behandelt und nach 1 Std. bei -20° erneut mit 1.7 ml des Grignard-Reagens versetzt. Nach weiteren 2 1/2 Std. bei -20° wurde mit Ammoniumchloridlösung versetzt und mit Methylenchlorid extrahiert. Die Extrakte wurden getrocknet und eingeengt. Der Rückstand wurde aus t-Butylmethylether/Hexan kristallisiert und das Kristallisat getrocknet, wobei man ebenfalls 1-[2(R)-[1(R)-(Benzyloxycarbamoyl)-2-(3,4,4-trimethyl-2,5-dioxo-1-imidazolidinyl)ethyl]-3-cyclopentylpropionyl)piperidin VII, Smp. 135-137° erhielt.

### Beispiel 15

Zur Debenzylierung wurde eine Suspension von 5.5 g 1-[2(R)-[1(R)-(Benzyloxycarbamoyl)-2-(3,4,4-trimethyl-2,5-dioxo-1-imidazolidinyl)ethyl]-3-cyclopentylpropionyl)piperidin VII in 40 ml Ethanol und 1.7 g Pd/C (5%) bei 22° /1 bar während 4 h hydriert. Die Suspension wurde filtriert, das Filtrat vollständig eingeengt und der Rückstand aus Wasser kristallisiert wobei man 3.9 g (85%) reines 1-[3-Cyclopentyl-2(R)-[1(R)-(hydroxycarbamoyl)-2-(3,4,4-trimethyl-2,5-dioxo-1-imidazolidinyl)ethyl]propionyl]piperidin I erhielt, MS (EI): 436 (40%).

### Beispiel 16

Zu einer Suspension von 2.11 g 1-[2(R)-[1(R)-Carboxy-2-(3,4,4-trimethyl-2,5-dioxo-1-imidazolidinyl)ethyl]-3-cyclopentylpropionyl)piperidin aus Beispiel 11 und 0.61 g N-Hydroxy-2-pyridon in 21 ml Methylenchlorid wurde bei 22° 0.78 g 2-Morpholino-ethylisocyanid gegeben und 3 Std. gerührt. Die Lösung wurde mit 0.58 g O-Trimethylsilyl-hydroxylamin versetzt und 2 Std. gerührt. Das Reaktionsgemisch wurde mit gesättigter NaHCO₃-Lösung und mit Wasser gewaschen und eingedampft. Der Rückstand wurde in 20 ml t-Butylmethylether und 0.23 ml Wasser gelöst, 1 1/2 Std. bei 22° gerührt, die Suspension mit 10 ml Hexan verdünnt, abfiltriert und der Rückstand bei 22°/11 mbar getrocknet wobei man 1.82 g (83%) reines 1-[3-Cyclopentyl-2(R)-[1(R)-(hydroxycarbamoyl)-2-(3,4,4-trimethyl-2,5-dioxo-1-imidazolidinyl)ethyl]propionyl]piperidin I erhielt, MS (EI): 436 (40%).

## Patentansprüche

1. Verfahren zur Herstellung einer Verbindung der Formel I 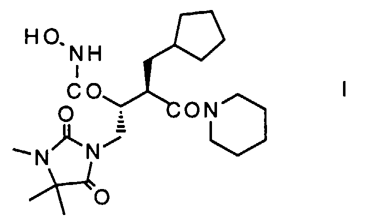 gekennzeichnet durch die folgenden Schritte:
a) Umsetzung einer Verbindung der Formel II 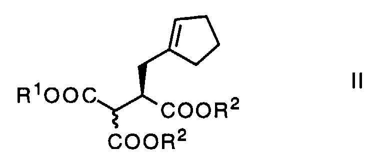 worin R² eine hydrogenolytisch abspaltbare Gruppe und
R¹ Methyl, Ethyl, Propyl, Butyl, Pentyl, Hexyl oder t-Butyl bedeutet,
mit 3-Brommethyl-1,5,5-trimethylhydantoin in Gegenwart einer Base zu einer Verbindung der Formel III 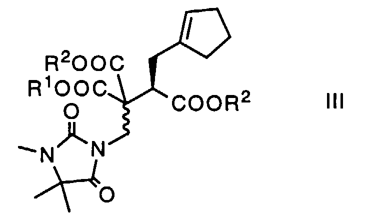 worin R¹ und R² die Bedeutungen wie vorstehend definiert haben,
b) Hydrogenolyse der Verbindung III in Gegenwart eines Metallkatalysators, nachfolgende Decarboxylierung in Gegenwart einer Base und Reinigung des so erhaltenen Produktes durch Zugabe einer weiteren Base, die zur Salzbildung geeignet ist, wobei eine Verbindung der Formel IV 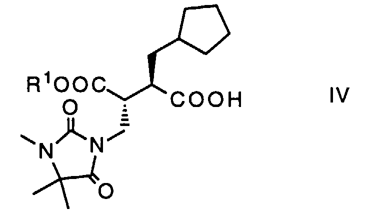 als Salz erhalten wird, worin R¹ wie oben definiert ist,
c) Umsetzung einer Verbindung der Formel IV mit Piperidin unter Aktivierung der Carbonsäure zu einer Verbindung der Formel V 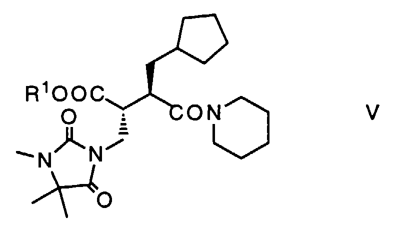 worin R¹ die oben angegebene Bedeutung hat,
d) Umsetzung einer Verbindung der Formel V,
worin R¹ Methyl, Ethyl, Propyl, Butyl, Pentyl oder Hexyl bedeutet,
mit einem mittels eines Alkylmagnesiumhalogenids aktivierten Benzylhydroxylaminhydrochlorids zur Verbindung VII 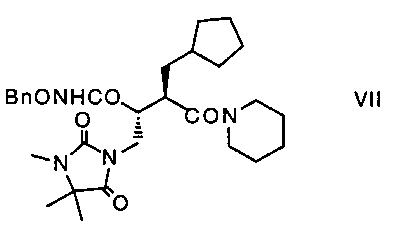 und hydrogenolytische Abspaltung der Benzylgruppe zur Verbindung I, oder
e) Abspaltung der t-Butylgruppe in einer Verbindung der Formel V, in der R¹ tert-Butyl bedeutet, mit einer Mischung aus einer Mineralsäure und einer Carbonsäure und/oder einem Carbonsäureester,
beziehungsweise Abspaltung der Estergruppe einer Verbindung der Formel V, in der R¹ Methyl, Ethyl, Propyl, Butyl, Pentyl oder Hexyl bedeutet, mit einem Alkali- oder Erdalkalimetallhydroxid
zur Verbindung VI 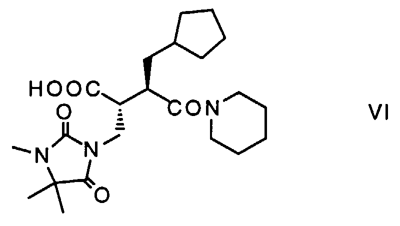 und entweder
- anschliessende Umsetzung der Verbindung VI mit Benzylhydroxylaminhydrochlorid unter Zusatz eines Aktivierungsmittels zur Verbindung VII, und hydrogenolytische Abspaltung der Benzylgruppe in der Verbindung VII, wobei Verbindung I erhalten wird, oder
- Umsetzung der Verbindung VI mit Trimethylsilyl-hydroxylamin oder Tetrahydropyranyl-hydroxylamin und Abspaltung der Trimethylsilyl- bzw. Tetrahydropyranyl-Gruppe nach an sich bekannten Methoden, wobei ebenfalls Verbindung I erhalten wird.

2. Verfahren zur Herstellung einer Verbindung der allgemeinen Formel II 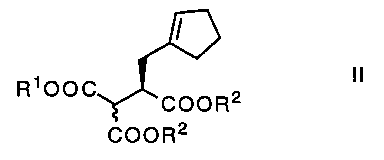 worin
R¹ Methyl, Ethyl, Propyl, Butyl, Pentyl, Hexyl oder tert-Butyl und
R² eine hydrogenolytisch abspaltbare Gruppe bedeuten,
gekennzeichnet durch die folgenden Schritte:
a) Umsetzung eines Glyoxylsäurederivates der Formel VIII 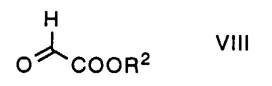 mit Methylencyclopentan in Gegenwart von katalytischen Mengen eines (R)-Binaphthyloxytitanium-Katalysators zu einer Verbindung der Formel IX 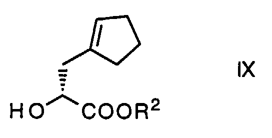 wobei R² eine hydrogenolytisch abspaltbare Gruppe darstellt,
b) Aktivierung einer Verbindung der Formel IX mit einem Sulfonsäurehalogenid R³-SO₂-X, worin R³ Trifluormethyl oder gegebenenfalls mit Nitro oder Halogen substituiertes Phenyl, und X Halogen bedeuten,
zu einer Verbindung der Formel X 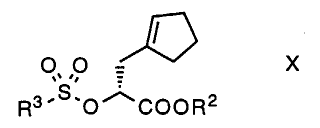 worin R² und R³ die vorstehend angegebenen Bedeutungen haben, und
c) Umsetzung einer Verbindung der Formel X mit einem Malonsäurederivat der Formel XI in Gegenwart einer Base
R¹O₂CCH₂CO₂R² XI
worin R² die vorstehende Bedeutung hat und
R¹ Methyl, Ethyl, Propyl, Butyl, Pentyl, Hexyl oder t-Butyl bedeutet,
zu einer Verbindung der Formel II.

3. Verfahren gemäss Anspruch 1 oder Anspruch 2, dadurch gekennzeichnet, dass R² Benzyl ist.

4. Verfahren gemäss Anspruch 2 oder Anspruch 3, dadurch gekennzeichnet, dass das Sulfonsäurehalogenid ortho-Nitrosulfonsäurechlorid ist.

5. Verbindungen der Formel IX, insbesondere (R)-3-Cyclopenten-1-enyl-2-hydroxy-propionsäurebenzylester.

6. Verbindungen der Formel X, insbesondere (R)-3-Cyclopent-1-enyl-2-(2-nitro-phenylsulfonyloxy)-propionsäurebenzylester.

7. Verbindungen der Formel II, insbesondere
(2R,3R)- und (2S,3R)-2-tert-Butyloxycarbonyl-3-cyclopent-1-enylmethyl-bernsteinsäuredibenzylester und
(2R,3R)- und (2S,3R)-2-Methyloxycarbonyl-3-cyclopent-1-enylmethyl-bernsteinsäuredibenzylester.

8. Verbindungen der Formel III, insbesondere
(2R,3R)- und (2S,3R)-2-tert-Butoxycarbonyl-3-cyclopent-1-enylmethyl-2-(3,4,4-trimethyl-2,5-dioxo-imidazolidin-1-yl-methyl)-bernsteinsäuredibenzylester und
(2R,3R)- und (2S,3R)-2-Methyloxycarbonyl-3-cyclopent-1-enylmethyl-2-(3,4,4-trimethyl-2,5-dioxo-imidazolidin-1-yl-methyl)-bernsteinsäuredibenzylester.

9. Verbindungen der Formel IV, insbesondere
4-tert-Butyl-2(R)-(cyclopentylmethyl)-3(R)-[(3,4,4-trimethyl-2,5-dioxo-1-imidazolidinyl)methyl]-bernsteinsäure und
4-Methyl-2(R)-(cyclopentylmethyl)-3(R)-[(3,4,4-trimethyl-2,5-dioxo-1-imidazolidinyl)methyl]-bernsteinsäure und deren Salze mit (+) Pseudoephedrin oder Ethanolamin.

10. Verbindungen der Formel V, insbesondere
1-[2(R)-[1(R)-(Methoxycarbonyl)-2-(3,4,4-trimethyl-2,5-dioxo-1-imidazolidinyl)ethyl]-3-cyclopentylpropionyl]piperidin und
1-[2(R)-[1(R)-(tert-Butoxycarbonyl)-2-(3,4,4-trimethyl-2,5-dioxo-1-imidazolidinyl)ethyl]-3-cyclopentylpropionyl]piperidin.

11. 1-[2(R)-[1(R)-Carboxy-2-(3,4,4-trimethyl-2,5-dioxo-1-imidazolidinyl) ethyl]-3-cyclopentylpropionyl)piperidin.

12. 1-[2(R)-[1(R)-(Benzyloxycarbamoyl)-2-(3,4,4-trimethyl-2,5-dioxo-1-imidazolidinyl)ethyl]-3-cyclopentylpropionyl)piperidin.

13. Verbindungen der Formel IX, insbesondere (R)-3-Cyclopenten-1-enyl-2-hydroxy-propionsäurebenzylester.

14. Verbindungen der Formel X, insbesondere (R)-3-Cyclopent-1-enyl-2-(2-nitro-phenylsulfonyloxy)-propionsäurebenzylester.

15. Verwendung einer Verbindung gemäss einem der Ansprüche 5-14 zur Herstellung der Verbindung der Formel I.
